# FASCICULE DE BREVET EUROPEEN

(11) **EP 0 376 807 B1**
(45) Date de publication et mention de la délivrance du brevet: **02.03.1994**
(21) Numéro de dépôt: 89403585.6
(22) Date de dépôt: 20.12.1989
(51) Int. Cl.: A61B 6/04

(54) **Table de support patient pour examens sur installations de scintigraphie**
Patientenliegetisch für Untersuchungen auf Szintigraphieanordnungen
Patient support table for examinations on scintigraphy apparatuses

(30) Priorité: 26.12.1988 FR 8817182
(43) Date de publication de la demande: 04.07.1990
(73) Titulaire: SOPHA MEDICAL, F-75008 Paris (FR)
(72) Inventeur: Pare, Christian, F-75116 Paris (FR); Fleury, Christophe, F-75116 Paris (FR)
(74) Mandataire: Ballot, Paul Denis Jacques

(56) Documents cités:
- EP-A- 0 288 698
- FR-A- 2 213 071
- US-A- 3 627 250
- US-A- 4 012 636

## Description

L'invention concerne les installations d'examen du corps humain qui utilisent différents rayonnements, notamment un rayonnement X, un rayonnement gamma etc... Elle concerne plus particulièrement, dans ces installations, les tables prévues pour supporter le patient tout en permettant l'examen de ce dernier par des instruments qui peuvent prendre toutes positions appropriées autour de lui.

Dans les installations d'examen radiologique ou scintigraphique le patient à examiner est allongé sur un lit en matériau transparent au rayonnement utilisé pour l'examen, par exemple des fibres de carbone. Ce lit est monté sur un piédestal qui comporte généralement différents dispositifs électromécaniques pour déplacer le lit suivant trois axes orthogonaux, deux axes horizontaux X′X et Y′Y et un axe vertical Z′Z. Les appareils qui sont utilisés pour l'examen proprement dit sont supportés par des bras animés de divers mouvements, ces divers mouvements ayant pour but d'amener les appareils d'examen à proximité du patient et de leur permettre d'effectuer une exploration du volume représenté par le patient.

On comprend que cette exploration ne doit pas être gênée par un quelconque obstacle, notamment le piédestal de la table de support. C'est pour cette raison que, dans certaines installations, le lit est monté en porte-à-faux par rapport au piédestal, le patient étant allongé sur la partie en porte-à-faux.

Dans un certain nombre d'installations, les appareils d'examen sont montés, du côté de la partie en porte-à-faux, sur un étrier ou arceau qui est porté par un bras tournant autour d'un axe horizontal parallèle à l'axe longitudinal de la table suivant des circonférences de diamètres variables. Le bras est porté par une colonne verticale qui peut se déplacer perpendiculairement par rapport à l'axe longitudinal de la table, ce qui permet de dégager la partie en porte-à-faux de la table pour mettre en place ou retirer le patient ou avoir accès à celui-ci en dehors des périodes d'examen proprement dit.

Une telle installation est particulièrement bien adaptée pour effectuer un examen tomographique du patient qui implique un mouvement circulaire, elliptique ou composite des appareils autour du lit. Elle est moins bien adaptée pour effectuer un examen longitudinal du patient, au-dessus et en-dessous, car le bras ne peut se déplacer longitudinalement; il est alors nécessaire de déplacer longitudinalement la table de support, ce qui présente certains inconvénients. D'abord, ce déplacement est désagréable pour le patient; ensuite, il implique que le lit en porte-à-faux supportant le patient pénètre dans la colonne verticale supportant le bras et qui, par conséquent, est percé d'un tunnel. Cela a comme autre inconvénient qu'il nécessite une place disponible au sol qui est égale à deux fois la longueur du lit.

Aussi, pour effectuer un examen du corps du patient sur toute sa longueur, on peut avoir recours à une autre méthode qui consiste à utiliser un deuxième lit que l'on place perpendiculairement au premier dont la partie en porte-à-faux a été éloignée du bras. Ce deuxième lit est porté par un support longitudinal qui est solidaire d'un pied longitudinal s'étendant au sol sous le lit. Par cette disposition particulière, l'appareil d'examen, porté par le bras, peut effectuer une exploration sous le lit du patient par déplacement de la colonne de support sur le rail disposé parallèlement au lit.

Un tel agencement présente l'inconvénient majeur d'utiliser deux lits encombrants et de provoquer des pertes de temps pour présenter sous la machine respectivement un lit ou un autre selon les types d'examen à effectuer.

Par ailleurs, on connaît par EP-A-0 288 698 une installation de radiologie, échographie et lithotripsie comprenant une table de support de patient constituée d'un lit monté mobile suivant au moins une direction parallèle à un axe horizontal sur un piédestal, et un support d'au moins un détecteur radiologique monté sur une colonne, la table de support et le lit pouvant prendre au moins deux positions angulaires différentes autour d'un axe vertical, de façon que, dans une première position, le lit soit placé dans le champ de l'appareil de radiographie, et que, dans une seconde position, le lit soit sortie du champ de l'appareil de radiographie, en vue d'un traitement par lithotripsie. Mais la colonne de support du détecteur radiologique est fixée au sol, de sorte que bien que l'émetteur de rayons X et le détecteur radiologique soient montés aux extrémités d'un bras cintré pouvant pivoter autour de l'axe longitudinal du lit sur un support lui-même monté pivotant autour d'un axe horizontal sur un plateau rotatif autour d'un axe vertical sur la colonne, pour permettre d'effectuer des examens tomographiques, il n'est malheureusement pas possible d'effectuer des examens corps entier du patient.

Le but de la présente invention est donc de réaliser une table de support de patient qui permet d'effectuer soit un examen tomographique, soit un examen corps entier.

L'invention se rapporte à une installation de radiologie ou scintigraphie comprenant une table de support de patient qui est constituée d'un lit monté mobile suivant au moins une direction parallèle à un axe horizontal OY sur un piédestal et un statif de support d'au moins un détecteur scintigraphique ou radiologique monté sur une colonne, ladite table de support pouvant prendre au moins deux positions angulaires différentes autour d'un axe parallèle à un axe vertical OZ perpendiculaire à l'axe horizontal OY, caractérisée en ce que ladite colonne est mobile suivant une direction parallèle à un autre axe horizontal OX perpendiculaire à l'axe horizontal OY, et ledit piédestal est mobile sur un rail au sol, disposé parallèlement au lit et pouvant également prendre lesdites positions angulaires différentes autour dudit axe vertical, afin de donner sa position angulaire à ladite table de support, ledit axe vertical étant disposé de sorte que le lit puisse rester dans le champ du détecteur lors du déplacement de la colonne de support du détecteur selon l'autre axe horizontal OX.

Dans une réalisation préférée, la première position angulaire correspond à un rail parallèle à l'axe OY de manière à permettre au statif d'effectuer un examen tomographique tandis que la deuxième position correspond à un rail parallèle à l'axe OX de manière à permettre au statif d'effectuer un examen corps entier par simple déplacement de la colonne.

En outre, il est prévu que les différentes positions angulaires soient déterminées par des butées au sol qui coopèrent avec le rail.

D'autres caractéristiques et avantages de la présente invention apparaîtront à la lecture de la description suivante d'un exemple particulier de réalisation, ladite description étant faite en relation avec le dessin joint dans lequel :
- la figure 1 représente une vue en perspective d'une installation d'examen radiologique ou scintigraphique comportant une table de support selon l'invention, en position d'examen tomographique, et
- la figure 2 représente une vue similaire à celle de la figure 1 mais avec une position de la table de support en position d'examen du corps entier.

L'appareil de radiologie ou scintigraphie selon l'invention comprend une table d'examen 10 et un dispositif de déplacement 11 du (ou des) détecteur(s) scintigraphique(s) ou radiologiques appelé(s) statif. La table d'examen 10 comporte un lit 12 monté sur un piédestal 13. Le lit 12 et le piédestal 13 comportent des moyens connus et non représentés qui coopèrent entre eux pour déplacer le lit suivant trois axes orthogonaux OX, OY et OZ représentés par le trièdre 14. Le piédestal 13 est monté coulissant sur un rail 15 qui est fixé au sol parallèlement à l'axe horizontal OY, ce qui permet le déplacement de la table suivant ledit axe (flèche 33) à l'aide de moyens portés par le piédestal et non représentés car ils sont connus par ailleurs.

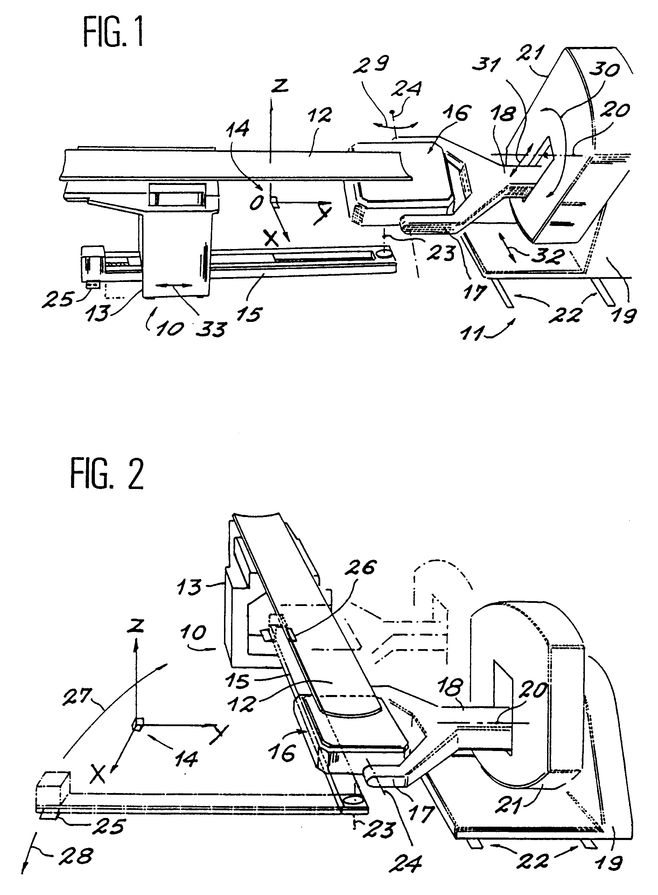

Dans l'exemple particulier de réalisation des figures 1 et 2, le statif 11 comprend un détecteur de rayons gamma, qui est monté pivotant (flèche 29) autour d'un axe 24 dans un étrier 17 à l'extrémité d'un bras 18. Ce bras 18 est également monté pivotant sur une colonne verticale 19 autour d'un axe 20 parallèle à OY par l'intermédiaire d'un support 21. Le support 21 tourne autour de l'axe 20 (flèche 30) tandis que l'extrémité du bras 18, côté support 21, se déplace (flèche 31) dans un plan vertical parallèle à l'axe OX de manière à modifier le rayon de rotation du bras. La colonne verticale 19 est montée coulissante sur des rails 22 à l'aide de moyens motorisés non représentés. Les rails 22 sont disposés de manière à permettre un déplacement de la colonne 19 suivant l'axe OX, perpendiculaire à l'axe OY (flèche 32).

A ce point de la description, on remarquera qu'un tel appareil permet d'effectuer un examen tomographique par la rotation du détecteur 16 autour de l'axe 20, le rayon du mouvement étant déterminé par la distance du bras 18 à cet axe. En déplaçant le lit dans la direction OY, on peut effectuer un examen tomographique d'une autre partie du corps. Cependant, dans une installation telle que celle qui apparaît sur la figure 1, un tel déplacement est limité par la longueur du bras 18. Aussi, dans certaines installations, le support 21 est percé d'un tunnel comme on l'a indiqué dans le préambule, ce qui permet d'effectuer un examen corps entier en déplaçant la table de manière continue. Une telle installation avec tunnel présente les inconvénients signalés dans le préambule.

Dans une installation sans tunnel comme celle de la figure 1, un examen corps entier est habituellement effectué en utilisant un deuxième lit spécial qui est disposé parallèlement à l'axe OX et qui permet le déplacement longitudinal sans entrave du détecteur 16 sous le lit par déplacement de la colonne 19 sur les rails 22.

L'invention propose une autre solution qui est de faire pivoter la table 10 de 90° de manière à amener le lit dans une direction parallèle à l'axe OX tout en restant dans le champ de l'instrument 16. La figure 2 montre cette nouvelle position de la table 10 par rapport au statif 11. La rotation de cette table est réalisée par l'intermédiaire du rail 15 qui est monté pivotant autour d'un axe vertical 23. Cet axe 23 est, par exemple, situé dans un plan vertical contenant l'axe 24 qui correspond à un axe médian du détecteur 16. Sa position exacte au sol est déterminée en fonction des limites extrêmes de déplacement de la colonne 19 sur les rails 22.

En effet, avec les positions respectives de la table 10 et du statif 11 telles que représentées sur la figure 2, l'examen corps entier est effectué par déplacement du statif 11 sur les rails 22 (position en pointillés de la figure 2). Il faut donc placer l'axe 23 pour qu'un tel examen soit possible sans déplacer le lit 12 par rapport au piédestal 13; il faut aussi que cette position permette les examens tomographiques dans les positions relatives indiquées à la figure 1.

Afin de permettre un changement de position précis de la table 10, les deux positions, à 90° l'une de l'autre, sont délimitées par des butées au sol référencées 25 pour celle de la figure 1 et 26 pour celle de la figure 2. Ces butées 25 et 26 sont évidemment des dispositifs d'arrimage du rail qui assurent une position fixe dans le temps.

Sur la figure 2, on a représenté un déplacement dans le sens de la flèche 27 mais on peut évidemment choisir un déplacement dans le sens de la flèche 28, auquel cas il faudrait prévoir une butée correspondante et éventuellement une autre position de l'axe 23.

Bien entendu, pour des applications autres que celles des figures 1 et 2, il serait possible de choisir d'autre positions angulaires différentes de 90° du rail 15 autour de son axe 23 selon les besoins et les commodités des examens à effectuer.

L'invention a été décrite dans son application à un appareil de scintigraphie mais elle peut également s'appliquer à des appareils de radiologie notamment.

## Revendications

1. Installation de scintigraphie ou radiologie, comprenant une table (10) de support de patient qui est constituée d'un lit (12) monté mobile suivant au moins une direction parallèle à un axe horizontal (OY) sur un piédestal (13) et un statif (11) de support d'au moins un détecteur scintigraphique (16) ou radiologique monté sur une colonne (19), ladite table de support (10) pouvant prendre au moins deux positions angulaires différentes autour d'un axe (23) parallèle à un axe vertical (OZ) perpendiculaire à l'axe horizontal (OY), caractérisée en ce que ladite colonne (19) est mobile suivant une direction parallèle à un autre axe horizontal (OX) perpendiculaire à l'axe horizontal (OY), et ledit piédestal (13) est mobile sur un rail (15) au sol, disposé parallèlement au lit (12) et pouvant également prendre lesdites positions angulaires différentes autour dudit axe (23) vertical, afin de donner sa position angulaire à ladite table de support (10), ledit axe (23) vertical étant disposé de sorte que le lit (12) puisse rester dans le champ du détecteur (16) lors du déplacement de la colonne (19) de support du détecteur (16) selon l'autre axe horizontal (OX).

2. Installation selon la revendication 1, caractérisée en ce que la première position angulaire correspond à un rail (15) parallèle à l'axe horizontal (OY) de manière à permettre au statif (11) d'effectuer un examen tomographique et en ce que la deuxième position correspond à un rail (15) parallèle à l'autre axe horizontal (OX) de manière à permettre au statif d'effectuer un examen corps entier par déplacement de la colonne (19).

3. Installation selon la revendication 1 ou 2, caractérisée en ce qu'elle comprend en outre au moins deux butées (25, 26) au sol déterminant les différentes positions angulaires du rail (15).

## Claims

1. Scintigraphy or radiology installation, comprising a patient support table (10) which is formed by a bed (12) mounted on a pedestal (13) for movement in at least one direction parallel with a horizontal axis (OY), and comprising a support stand (11) for at least one scintigraphic or radiological detector (16) mounted on a column (19), the said support table (10) being capable of adopting at least two different angular positions around an axis (23) parallel with a vertical axis (OZ) perpendicular to the horizontal axis (OY), characterised in that the said column (19) can move in a direction parallel with another horizontal axis (OX) perpendicular to the horizontal axis (OY), and the said pedestal (13) can move along a rail (15) on the ground, which rail is arranged parallel with the bed (12) and is also capable of adopting the said different angular positions around the said vertical axis (23) so as to give the said support table (10) its angular position, the said vertical axis (23) being arranged so that the bed (12) can remain within the field of the detector (16) during displacement of the support column (19) for the detector (16) along the other horizontal axis (OX).

2. Installation according to claim 1, characterised in that the first angular position corresponds to a rail (15) parallel with the horizontal axis (OY) so as to enable the stand (11) to perform a tomographic examination, and in that the second position corresponds to a rail (15) parallel with the other horizontal axis (OX) so as to enable the stand to perform a whole body examination by displacement of the column (19).

3. Installation according to claim 1 or 2, characterised in that it further comprises at least two limit stops (25, 26) on the ground which determine the different angular positions of the rail (15).

## Patentansprüche

1. Szintigraphie- oder Röntgenanlage mit einem Patientenliegetisch (10), der durch eine Auflage (12) gebildet ist, die auf einem Sockel (13) in wenigstens einer zu einer horizontalen Achse (OY) parallelen Richtung beweglich gelagert ist, und mit einem Ständer (11) als Träger wenigstens eines szintigraphischen oder röntgenologischen Detektors (16), der an einer Säule (19) montiert ist, wobei der Liegetisch (10) wenigstens zwei verschiedene Winkelstellungen um eine Achse (23) einnehmen kann, die zu einer zu der horizontalen Achse (OY) senkrechten Achse (OZ) parallel ist, dadurch gekennzeichnet, daß die Säule (19) in einer Richtung beweglich ist, die zu einer anderen, zu der horizontalen Achse (OY) senkrechten horizontalen Achse (OX) parallel ist, und daß der Sockel (13) auf einer Schiene (15) auf dem Boden beweglich ist, die parallel zu der Auflage (12) angeordnet ist und ebenfalls die verschiedenen Winkelstellungen um die vertikale Achse (23) einnehmen kann, um ihre Winkelstellung dem Liegetisch (10) zu erteilen, wobei die vertikale Achse (23) so angeordnet ist, daß die Auflage (12) bei der Verstellung der den Detektor (16) tragenden Säule (19) entlang der anderen horizontalen Achse (OX) im Feld des Detektors (16) bleiben kann.

2. Anlage gemäß Anspruch 1, daß die erste Winkelstellung einer zu der horizontalen Achse (OY) parallelen Schiene (15) entspricht, damit der Ständer (11) eine tomographische Untersuchung durchführen kann, und daß die zweite Winkelstellung einer zu der anderen horizontalen Achse (OX) parallelen Schiene (15) entspricht, damit der Ständer eine Ganzkörperuntersuchung durch Verstellung der Säule (19) durchführen kann.

3. Anlage gemäß Anspruch 1 oder 2, dadurch gekennzeichnet, daß sie außerdem wenigstens zwei Anschläge (25, 26) am Boden enthält, die die verschiedenen Winkelstellungen der Schiene (15) bestimmen.
